# EUROPEAN PATENT APPLICATION

(11) **EP 1 733 704 A2**
(43) Date of publication of application: **20.12.2006**
(21) Application number: 06252606.6
(22) Date of filing: 18.05.2006
(51) Int. Cl.: A61F 2/30, A61F 2/38, A61B 19/00, A61B 17/58, A61F 2/46

(54) **Orthopaedic prosthesis having a superparamagnetic material**

(30) Priority: 13.06.2005 US 151146
(71) Applicant: DePuy Products, Inc., Warsaw, IN 46581 (US)
(72) Inventor: Sherman, Jason T., Warsaw, IN 46582 (US); Disilvestro, Mark R., Columbia City, IN 46725 (US)
(74) Representative: Belcher, Simon James

(57) **Abstract**

An orthopaedic implant (10) such as an elongated nail or a joint prosthesis (10) includes a superparamagnetic material (22,24,26). The material can be provided in a hole in the implant (10), for example as a coating or at least partially filling the hole, or on an external surface of the implant close to the hole.

## Description

This invention relates generally to an orthopaedic prosthesis.

During the lifetime of a patient, it may be necessary to perform a orthopaedic procedure, such as a joint replacement procedure, on the patient as a result of, for example, disease or trauma. The orthopaedic procedure may involve the use of a prosthesis which is implanted into one or more of the patient's bones.

In one aspect, the invention provides an implantable orthopaedic prosthesis which includes a superparamagnetic material.

The prosthesis may include an elongated nail having a number of holes defined therein. The superparamagnetic material may be disposed in, around, or proximate to the holes.

The prosthesis may include a polyethylene implant.

The implantable orthopaedic prosthesis may include multiple components with one or more of such components having superparamagnetic material secured thereto.

The superparamagnetic material may be arranged in the form of a symbol, pattern, or any other type of indicia.

The prosthesis of the invention can be used in a method of determining the position of an orthopaedic prosthesis subsequent to implantation thereof, which includes the step of exposing the implanted orthopaedic prosthesis to a magnetic field to detect a superparamagnetic material secured to the prosthesis. In such a way, the relative position of one or more of the components of the prosthesis may be determined. Such a method may also be used to determine the position of the prosthesis relative to a bone. The method may also be used to determine the degree of wear of the prosthesis.

A suitable method of determining the position of a first implanted orthopaedic component relative to a second implanted orthopaedic component comprises the steps of:
exposing the first implanted orthopaedic component and the second implanted orthopaedic component to a magnetic field,
determining the position of a superparamagnetic material secured to the first implanted orthopaedic component relative to a superparamagnetic material secured to the second implanted orthopaedic component, and
correlating the position of the first implanted orthopaedic component relative to the second implanted orthopaedic component based on the determining step.

Another method of determining the position of an implanted orthopaedic component relative to a bone comprises the steps of:
exposing the implanted orthopaedic component and the bone to a magnetic field,
determining the position of a superparamagnetic material secured to the implanted orthopaedic component relative to a superparamagnetic material secured to the bone, and
correlating the position of the implanted orthopaedic component relative to the bone based on the determining step.

A further method of monitoring wear of a polyethylene orthopaedic component subsequent to implantation thereof comprises the steps of:
exposing the implanted polyethylene orthopaedic component to a magnetic field,
detecting a superparamagnetic material secured to the implanted polyethylene orthopaedic component, and
determining wear of the implanted polyethylene orthopaedic component based on the detecting step.

Embodiments of the invention will now be described by way of example with reference to the accompanying drawings, in which:
FIGS. 1 and 2 are cross sectional views of a femoral prosthesis implanted into the femur of a patient, note that the prosthesis is shown in elevation for clarity of description;
FIG. 3 is a perspective view of a knee prosthesis;
FIG. 4 is an elevational view of a knee prosthesis;
FIGS. 5 to 7 are fragmentary elevational views of an intramedullary nail; and
FIG. 8 is an elevational view of a tibial bearing.

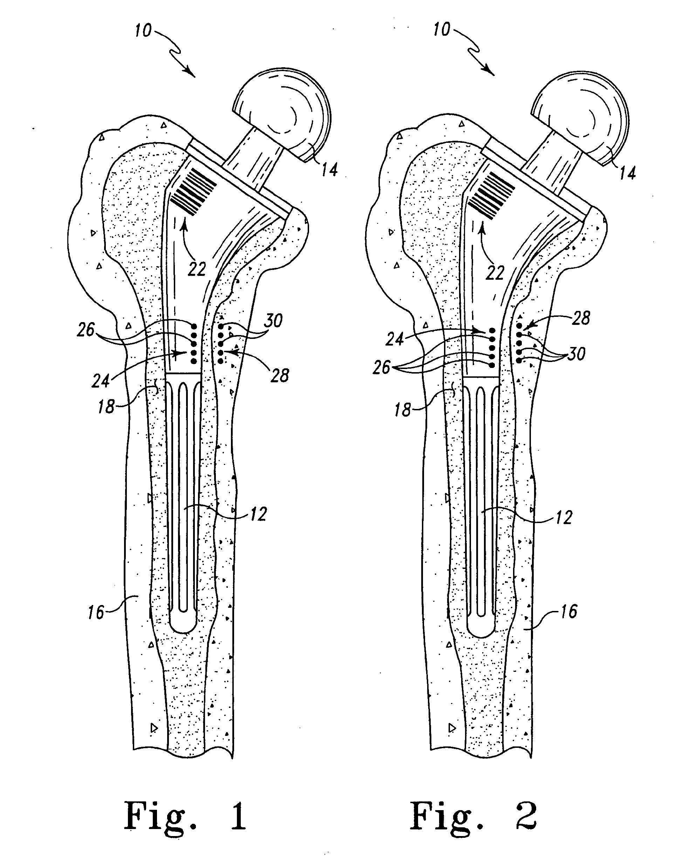

Referring to the drawings, FIGS. 1 to 8 show orthopaedic implants which include a superparamagnetic material. What is meant herein by the term "superparamagnetic material" is a material that (i) is not magnetic prior to being exposed to a magnetic field, but becomes magnetic during its exposure to the magnetic field, and (ii) exhibits a residual magnetism for a short period of time after being removed from the magnetic field. The superparamagnetic material may be made from any substance as long as the resulting material exhibits superparamagnetism. Examples of superparamagnetic materials include materials having a collection of particles where the magnetic domain of each particle is so small that the material exhibits the above described superparamagnetism. The size and number of particles making up the collection can vary as long as the collection is superparamagnetic. For example, particles having a grain size from about 0.2 to 1.5 microns may be included in the collection, although particles having a grain size outside of such a range may also be included provided such particles exhibit superparamagnetism. The particles may be made from any substance that will exhibit superparamagnetism. For example, the particles may be made from a ferromagnetic substance, such as Fe₃O₄ and Fe₂O₃. In addition, each particle in the collection may be made of the same substance, or the collection may be a mixture of particles made from different substances. The particles may be dispersed within a polymer or a ferrofluid encased by a polymer. In addition, the particles may be doped with metal ions other than iron ions such as Co, Mn, and/or Cr. Examples of suitable superparamagnetic particles, including details of compositions, doping materials, and methods of manufacture, are disclosed in US-4108787.

When a superparamagnetic material is exposed to a magnetic field, it produces distortion in the field that may be measurable by use of any of a number of techniques. For example, such distortion may be measurable with magnetic resonance imaging (MRI), or by the use of highly sensitive magnetic sensors such as Giant Magneto-Resistive (GMR), Magnetic Tunnel Junctions (MTJ), Spin Dependent Tunneling (SDT), Anisotropic Magneto-Resistive (AMR), Fluxgate Magnetometers (FGM), Superconducting Quantum Interference Devices (SQUIDs), or the like.

As will be described below in greater detail, superparamagnetic materials may be incorporated into the design of an implantable orthopaedic prosthesis to allow for the in vivo determination of a number of aspects of the prosthesis. For example, subsequent to implantation of the orthopaedic implant, superparamagnetic materials may be used to monitor wear of the implant, identify the implant, determine the position of the components of the implant relative to one another, determine the onset of subsidence or migration of the implant, visualize the implant (in the case of, for example, polyethylene implants), along with other uses.

Referring now in particular to FIGS. 1 and 2, there is shown an implantable femoral prosthesis 10 for implantation into a patient's femur during performance of a hip replacement procedure. It should be appreciated that although the concepts discussed in relation to FIGS. 1 and 2 are described in regard to a prosthesis for use in the performance of a hip replacement procedure, such concepts may be utilized in conjunction with a prosthesis for use in other orthopaedic procedures. For example, such concepts may be utilized in the construction of a prosthesis for implantation into the humerus, radius, ulna, tibia, fibula, spine, skull, or any of the metatarsals or metacarpals, including associated joint prostheses (for example, hip, shoulder, or knee prostheses).

The femoral prosthesis 10 includes a stem 12 and a head 14. The prosthesis 10 is configured to be implanted into the femur 16 of a patient in order to replace certain natural features of the patient's femur as a result of, for example, disease or trauma. The prosthesis 10 is implanted into a surgically prepared (for example, reamed and/or broached) medullary canal of the femur 16. The prosthesis is secured in place by the use of bone cement 18, although cement-less joints may be used.

The prosthesis 10 includes a superparamagnetic material arranged in a pattern 22. In the case of the prosthesis 10 shown in FIGS. 1 and 2, the pattern 22 defines an indicia such a machine-readable indicia. For example, amongst others, the pattern 22 may define barcode, such as a conventional "one-dimensional" barcode (as shown in FIG. 1), or a two-dimensional barcode such as, but not limited to, those which are referred to using the trade marks 3-DI, ArrayTag, Aztec Code, Small Aztec Code, Codablock, Code 1, Code 16K, Code 49, CP Code, DataGlyphs, Data Matrix, Datastrip Code, Dot Code A, hueCode, INTACTA.CODE, MaxiCode, MiniCode, PDF 417, Micro PDF417, QR Code, SmartCode, Snowflake Code, SuperCode, or Ultracode. Other types of machine-readable codes may also be used.

It should be appreciated that the barcode may be used as a key into a database containing detailed identification information about the prosthesis 10 such as the manufacturer, product line, lot number, serial number, size, implanting surgeon, etcetera. In addition to, or in lieu of, being a key into a database, the barcode may be a portable database with such implant identification information being encoded in the barcode itself. This may be particularly useful when two-dimensional barcodes are used, although use of the barcode as a portable database (as opposed to a key) is not limited to two-dimensional barcodes. Due to the relatively small size of the individual particles of the superparamagnetic material (for example, 0.2 - 1.5 microns), the pattern 22 (for example, the barcode) may be configured with a relatively high data density.

The superparamagnetic pattern 22 of the prosthesis 10 may be visualized, in vivo. Specifically, subsequent to implantation of the prosthesis 10, the patient may be exposed to a magnetic field, such as an MRI, an X-ray, and/or a custom measurement system utilizing one or more of the aforementioned highly sensitive magnetic sensors, to visualize the barcode (or other type of human-readable or machine-readable indicia used as the pattern 22). In such a way, information, such as implant identification information, may be encoded with the prosthesis 10.

The superparamagnetic pattern 22 may be placed (for example, coated) on the surface of the implant. Alternatively, the pattern may be embedded in the implant such as in the case of a polyethylene implant.

It should be appreciated that, in addition to the barcodes described herein, the pattern 22 may take the form of any type of machine-readable indicia. In addition, the pattern 22 may be embodied as a human-readable indicia such as one or more alphanumeric characters.

Although use of a superparamagnetic material in the formation of human-readable or machine-readable indicia is described in regard to the stem 12 of the femoral prosthesis 10, it should be appreciated that any type of prosthesis may utilize such an indicia. For example, such concepts may be utilized in conjunction with the other components of a hip prosthesis (for example, the head 14 or an acetabular cup and/or bearing), the components of a knee prosthesis (for example, a femoral component, tibial tray, and/or tibial bearing), the components of a shoulder prosthesis (for example, a humeral stem, head and/or glenoid bearing), long bone prosthesis (for example, distractors or trauma nails), spinal implants, or any other type of prosthesis.

The prosthesis 10 also includes another pattern 24 of superparamagnetic material. In the embodiment of FIGS. 1 and 2, the pattern 24 is embodied as a number of points 26, although other pattern configurations may be used. Each of the points 26 may be a single superparamagnetic particle or a number of particles. Any number of points 26 (including only a single point 26) may be used.

A pattern 28 of superparamagnetic material may be secured to the patient's femur 16. As with the pattern 24 of the prosthesis 10, in the embodiment of FIGS. 1 and 2, the pattern 28 of the femur 16 is also embodied as a number of points 30, although other pattern configurations may be used. Each of the points 30 may be a single superparamagnetic particle or a number of particles. Any number of points 30 (including only a single point 30) may be used. The pattern 28 of the femur 16 may include fewer or more points than the pattern 24 of the prosthesis.

The superparamagnetic patterns 24, 28 may be used, in vivo, to determine the position of the prosthesis 10 relative to the femur 16. Specifically, subsequent to implantation of the prosthesis 10, the patient may be exposed to a magnetic field. By measuring the distortion the superparamagnetic material causes in the applied magnetic field at one or more of the points 26, the in vivo position of the point(s) 26 may be determined by, for example, triangulation. Likewise, by measuring the distortion the superparamagnetic material causes in the applied magnetic field at one or more of the points 30, the in vivo position of the point(s) 30 may be determined by, for example, triangulation. Once the in vivo position of the point(s) 26 and the point(s) 30 have been determined, the position of the prosthesis 10 relative to the femur 16 may be correlated.

It should be appreciated that the position of the prosthesis 10 relative to the femur 16 may be monitored over time subsequent to implantation of the prosthesis 10. In such a way, anomalies, such a implant subsidence or migration, may be detected. For example, the alignment of each of the point(s) 26 of the prosthesis 10 relative to a corresponding point(s) 30 of the femur 10 may be noted at the time of implantation of the prosthesis 10 (for example, during or relatively soon after surgery). In the embodiment of FIG. 1, each of the points 26 is aligned substantially horizontally (as viewed in the orientation of the page) with a corresponding point 30. However, as shown in FIG. 2, subsidence of the prosthesis 10 may occur over time. In this case, the points 26 of the prosthesis 10 have shifted distally relative to the points 30 of the femur 16. By determining the position of the points 26 relative to the points 30, the position of the prosthesis 10 relative to the femur 16 may be correlated, and hence the degree of subsidence (or migration) may be determined.

It should be appreciated that although the points 26 of the prosthesis 10 are shown in FIG. 1 as being aligned horizontally with the points 30 of the femur 16, such an arrangement need not be the case. In particular, at the time of surgery, the points 26, 30 may be misaligned (that is, not arranged horizontally). The degree of such misalignment (for example, the distance) may serve as baseline measurement, with subsequent measurements being compared to such a baseline to determine post-operative movement of the prosthesis 10.

In addition to triangulation, other methods for determining the location of the point(s) 26 of the prosthesis 10 and the point(s) 30 of the femur 16 in response to exposure to a magnetic field may also be used. For example, custom models, along with the associated measurement scheme and calculations, may be used to characterize the magnetic field as a permanent magnet to determine the location(s) of the point(s) 26 of the prosthesis 10 and the point(s) 30 of the femur 16. Such models may be analytical, finite elemental, or based on any other type of suitable mathematical method.

Moreover, the superparamagnetic patterns 24, 28 may be visualized in a similar manner to as described above in regard to the pattern 22 (for example, by use of an MRI, an X-ray, and/or a custom measurement system utilizing one or more of the aforementioned highly sensitive magnetic sensors). The resultant film or digital rendering, (for example, digital X-ray) may then be analyzed manually (for example, visually) or by an automated device to determine the position of the point(s) 26 relative to the point(s) 30 with such data then being utilized to correlate the position of the prosthesis 10 relative to the femur 16.

As described above, although the use of a superparamagnetic material to monitor implant position/movement is described in detail in regard to the stem 12 of the femoral prosthesis 10, it should be appreciated that such concepts may be used to monitor implant position/movement of any type of prosthesis relative to a bone (or other anatomical structure). For example, such concepts may be used in regard to the other components of a hip prosthesis (for example, the head 14 or an acetabular cup and/or bearing), the components of a knee prosthesis (for example, a femoral component, tibial tray, and/or tibial bearing), the components of a shoulder prosthesis (for example, a humeral stem, head and/or glenoid bearing), long bone prostheses (for example, distractors or trauma nails), spinal implants, or any other type of prosthesis.

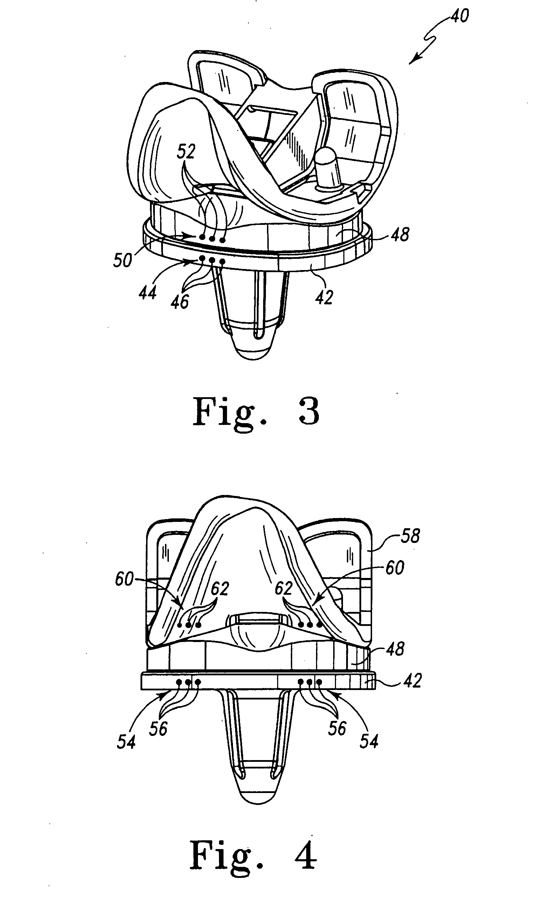

Patterns of superparamagnetic material may be used in other implant applications, as well. For example, as shown in FIGS. 3 and 4, the position of two or more of the components of a knee prosthesis 40 relative to one another may be determined by use of superparamagnetic material. It should be appreciated that although the concepts discussed in relation to FIGS. 3 and 4 are herein described in regard to a prosthesis for use in the performance of a knee replacement procedure, such concepts may be utilized in conjunction with a prosthesis for implantation into other joints of the body. For example, such concepts may be utilized in the construction of a hip or shoulder prosthesis, along with any other prosthesis for implantation into bones such as the femur, humerus, radius, ulna, tibia, fibula, spine, or any of the metatarsals or metacarpals.

As shown in FIG. 3, a tibial tray 42 of the knee prosthesis 40 has a pattern 44 of superparamagnetic material. In the embodiment of FIG. 3, the pattern 44 is embodied as a number of points 46, although other pattern configurations may be used. Each of the points 46 may be a single superparamagnetic particle or a number of particles. Any number of points 46 (including only a single point 46) may be used.

A tibial bearing 48 of the knee prosthesis 40 has a pattern 50 of superparamagnetic material. As with the pattern 44 of the tibial tray 42, in the embodiment of FIG. 3, the pattern 50 of the tibial bearing 48 is also embodied as a number of points 52, although other pattern configurations may be used. Each of the points 52 may be a single superparamagnetic particle or a number of particles. Any number of points 52 (including only a single point 52) may be used. The pattern 50 of the tibial bearing 48 may include fewer or more points than the pattern 44 of the tibial tray 42.

The superparamagnetic patterns 44, 50 may be used, in vivo, to detect motion (for example, micromotion) of the tibial bearing 48 relative to the tibial tray 42. Specifically, subsequent to implantation of the knee prosthesis 40, the patient may be exposed to a magnetic field. By measuring the distortion the superparamagnetic material causes in the applied magnetic field at one or more of the points 46 and one or more of the points 52 (for example, by triangulation of such points 46, 52) as a function of time, motion of the pattern 50 of the tibial bearing 48 relative to the pattern 44 of the tibial tray 42 may be detected. Such motion of the patterns 44, 50 may be correlated to motion of the components (for example, the tibial bearing 48 and the tibial tray 42).

It should be appreciated that although the points 46 of the tibial tray 42 are shown in FIG. 3 as being aligned vertically with the points 52 of the tibial bearing 48, such an arrangement need not be the case. In particular, at the time of surgery, the points 46, 52 may be misaligned (that is, not aligned vertically). The degree of such misalignment (for example, the distance) may serve as baseline measurement, with subsequent measurements being compared to such a baseline to determine post-operative motion of the components of the knee prosthesis 40.

In addition to triangulation, other methods for detecting the motion between of the point(s) 52 of the tibial bearing 48 and the point(s) 46 of the tibial tray 42 may also be used. For example, custom models, along with the associated measurement scheme and calculations, may be used to characterize the magnetic field as a permanent magnet to detect motion between of the point(s) 52 of the tibial bearing 48 and the point(s) 46 of the tibial tray 42. Such models may be analytical, finite elemental, or based on any other type of suitable mathematical method. Moreover, the location of the point(s) 52 of the tibial bearing 48 and the point(s) 46 of the tibial tray 42 may be visualized over time by use of, for example, an MRI, an X-ray, and/or a custom measurement system utilizing one or more of the aforementioned highly sensitive magnetic sensors. Such location over time data may be correlated to motion.

Although use of a superparamagnetic material to monitor motion between the components of an implant is described in detail in regard to a tibial bearing and a tibial tray, it should be appreciated that such concepts may be used to monitor motion of any of the components of any type of prosthesis. For example, such concepts may be used in regard to the other components of a knee prosthesis (for example, motion of the tibial tray or bearing relative to the femoral component), the components of a hip prosthesis (for example, a femoral implant, head, acetabular cup, and/or bearing), the components of a shoulder prosthesis (for example, a humeral stem, head and/or glenoid bearing), the components of a long bone prosthesis (for example, distractors), spinal implants, or any other type of prosthesis.

As shown in FIG. 4, the tibial tray 42 of the knee prosthesis 40 may have one or more patterns 54 of superparamagnetic material. In the embodiment of FIG. 4, each of the patterns 54 is embodied as a number of points 56, although other pattern configurations may be used. Each of the points 56 may be a single superparamagnetic particle or a number of particles. Any number of points 56 (including only a single point 56) may be used. Any number of patterns 54 may be used.

A femoral component 58 of the knee prosthesis 40 has one or more patterns 60 of superparamagnetic material. Similar to the pattern 54 of the tibial tray 42, in the embodiment of FIG. 4, each of the patterns 60 of the tibial bearing 48 is also embodied as a number of points 62, although other pattern configurations may be used. Each of the points 62 may be a single superparamagnetic particle or a number of particles. Any number of points 62 (including only a single point 62) may be used. Any number of patterns 60 may be used. The pattern 60 of the femoral component 58 may include fewer or more points than the pattern 54 of the tibial tray 42.

The superparamagnetic patterns 54, 60 may be used, in vivo, to determine the position of the femoral component 58 relative to the tibial tray 42. In such a way, post-operative wear of the tibial bearing 48 may be monitored (such bearings generally being constructed of a polymer). In particular, as the tibial bearing 48 wears, the distance between the femoral component 58 and the tibial tray 42 decreases since such a distance is indicative of the thickness of the tibial bearing 48. By monitoring the position of the superparamagnetic patterns 54, 60 relative to one another (and hence the position of the femoral component 58 and the tibial tray relative to one another), the thickness of the tibial bearing may be monitored. Subsequent to implantation of the knee prosthesis 40, the patient may be exposed to a magnetic field. By measuring the distortion the superparamagnetic material causes in the applied magnetic field at one or more of the points 56 and one or more of the points 62 (for example, by triangulation of such points 56, 62) the location of the pattern 60 of the femoral component 58 relative to the pattern 54 of the tibial tray 42 may be determined. The location of the patterns 54, 60 may be correlated to a distance between the components (that is, the femoral component 58 and the tibial tray 42) and hence the thickness of the tibial bearing 48.

It should be appreciated that although the points 56 of the tibial tray 42 are shown in FIG. 4 as being aligned vertically with the points 62 of the femoral component 58, such an arrangement need not be the case. In particular, at the time of surgery, the points 56, 62 may be misaligned (that is, not aligned vertically). The degree of such misalignment may be noted and compensated for in subsequent measurements of the knee prosthesis 40.

In addition to triangulation, other methods for determining the distance between of the point(s) 62 of the femoral component 58 and the point(s) 56 of the tibial tray 42 may also be used. For example, custom models, along with the associated measurement scheme and calculations, may be used to characterize the magnetic field as a permanent magnet to determine the distance between the point(s) 62 of the femoral component 58 and the point(s) 56 of the tibial tray 42. Such models may be analytical, finite elemental, or based on any other type of suitable mathematical method.

Although the use of a superparamagnetic material to monitor wear of a knee bearing (that is, the tibial bearing 48) is herein described in detail, it should be appreciated that such concepts may be used to monitor wear of any of the components of any type of prosthesis. For example, such concepts may be used in regard to the other components of a knee prosthesis, the components of a hip prosthesis (for example, an acetabular bearing), the components of a shoulder prosthesis (for example, a glenoid bearing), any of the components of a long bone prosthesis, spinal implants, or any other type of prosthesis.

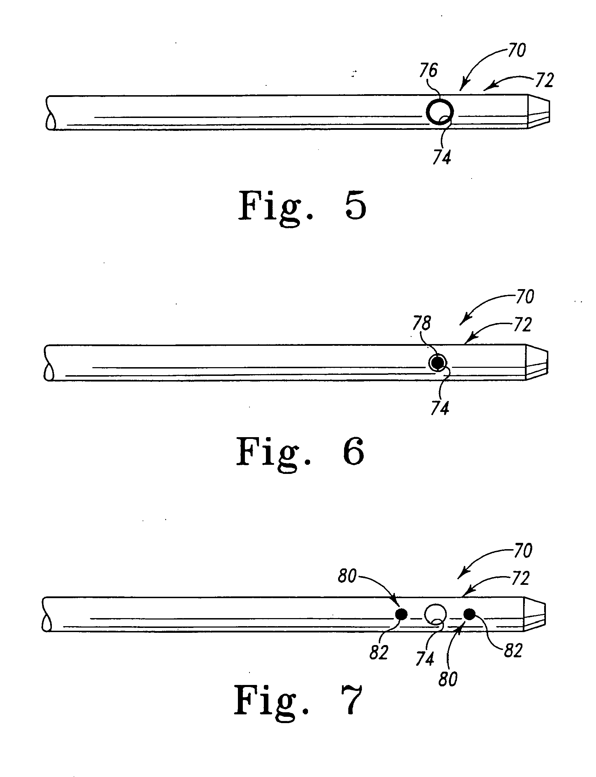

Superparamagnetic material may be used in other implant applications, as well. For example, as shown in FIGS. 5 to 7, the in vivo position of one or more holes (for example, screw holes) of an intramedullary nail 70 may be determined by use of superparamagnetic material. It should be appreciated that although the concepts discussed in relation to FIGS. 5 to 7 are described in relation to an intramedullary nailing procedure, such concepts may be utilized in conjunction with a prosthesis for implantation into any bone or joint of the body. For example, such concepts may be utilized in conjunction with a knee, hip, or shoulder prosthesis, along with any other prosthesis for implantation into bones such as the femur, humerus, radius, ulna, tibia, fibula, spine, skull, or any of the metatarsals or metacarpals. Moreover, although the concepts discussed in relation to FIGS. 5 to 7 are described in relation to the in vivo location of holes (for example, screw holes), such concepts may be used for the in vivo location of any feature of a prosthesis.

As shown in FIGS. 5 to 7, a distal end portion 72 of the intramedullary nail 70 has a screw hole 74 defined therein. During implantation, the nail 70 is implanted into the intramedullary canal of one of the patient's long bones. Once implanted, a bone screw (not shown) is advanced through the cortical bone on one side of the bone, through the screw hole 74, and into or through the cortical bone on the opposite side of the bone. The nail 70 may be embodied with any number, size, and/or orientation (for example, angle) of screw holes 74. Superparamagnetic material may be used to identify the location of the screw holes 74 in vivo thereby eliminating the need for direct visualization or repeated use of fluoroscopy.

For example, as shown in the embodiment of FIG. 5, an amount of superparamagnetic material may be placed around the screw hole 74. For example, the superparamagnetic material may be disposed in a coating 76 that is placed on the outer surface of the nail 70 in an annular pattern around the periphery of the screw hole 74. Alternatively, or in addition to, the superparamagnetic coating 76 may be disposed on the walls of the nail 70 that define the hole 74.

As shown in FIG. 6, the screw hole 74 may be filled (or partially filled) with superparamagnetic material. One way to do so is by disposing the superparamagnetic material in a gel-like substance 78. The gel-like substance 78 may entirely fill the screw hole 74, although only a portion of the screw hole 74 need be filled.

As shown in FIG. 7, one or more patterns 80 of superparamagnetic material may disposed on the outer surface of the nail 70 proximate to the screw hole 74. In the embodiment of FIG. 7, one of a pair of patterns 80 is disposed on opposite sides of the screw hole 74. In the described embodiment, each of the patterns 80 is embodied as a point 82, although other pattern configurations may be used. Each of the points 82 may be a single superparamagnetic particle or a number of particles. Any number of points 82 may be used.

The superparamagnetic material may be used, in vivo, to determine the position of the screw hole 74 of the nail 70. In such a way, a bone screw can be installed in the screw hole 74 without direct visualization of the hole. To do so, subsequent to implantation of the nail 70 into the intramedullary canal of one of the patient's long bones, the patient may be exposed to a magnetic field. By measuring the distortion the superparamagnetic material causes in the applied magnetic field at (for example, by triangulation) the location of the coating 76 (in the case of FIG. 5), the gel-like substance 78 (in the case of FIG. 6), or the pattern(s) 80 may be detected. The location of the screw holes 74 may then be correlated from the location of the coating 76, gel-like substance 78, or pattern(s) 80. The bone screws may then be installed (for example, percutaneously installed) in the screw holes 74.

In addition to triangulation, other methods of determining the location of the coating 76, the gel-like substance 78, and/or the pattern(s) 80 may also be used. For example, custom models, along with the associated measurement scheme and calculations, may be used to characterize the magnetic field as a permanent magnet to determine the location of the coating 76, the gel-like substance 78, and/or the pattern(s) 80. Such models may be analytical, finite elemental, or based on any other type of suitable mathematical method.

Although the use of a superparamagnetic material to locate features of an implant is herein described in regard to the screw holes of an intramedullary nail, it should be appreciated that such concepts may be used to locate holes in other types of prosthesis or to locate features other than holes on the nail or other types of prosthesis. For example, such concepts may be used to locate features on the components of a knee prosthesis, a hip prosthesis, a shoulder prosthesis, other long bone prostheses (for example, distractors), spinal implants, or any other type of prosthesis.

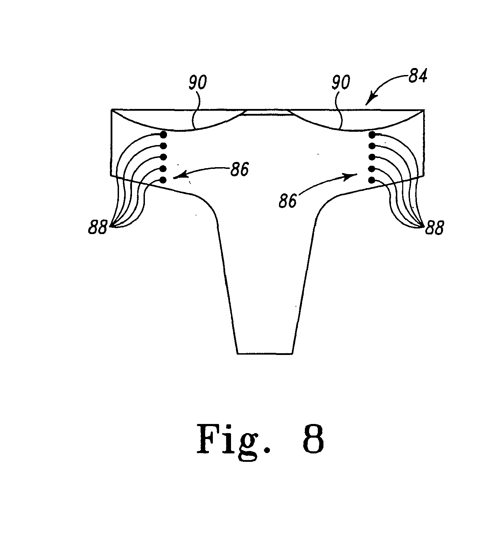

Referring now to FIG. 8, there is shown a tibial bearing 84. The tibial bearing 84 has one or more patterns 86 of superparamagnetic material. In the exemplary embodiment of FIG. 8, each of the patterns 86 is embodied as a number of points 88, although other pattern configurations may be used. Each of the points 88 may be a single superparamagnetic particle or a number of particles. Any number of points 88 may be used. Any number of patterns 86 may be used. In the exemplary embodiment described herein, one of the patterns 86 is embedded in the polymer tibial bearing 84 so as to extend in the distal direction away from of each of a pair bearing surfaces 90 on which the condyles (not shown) of a natural or prosthetic femur bear.

The superparamagnetic patterns 86 may be used, in vivo, to determine the degree of wear of the tibial bearing 84. For example, subsequent to implantation of the tibial bearing 84, the patient may be exposed to a magnetic field. By detecting the distortion the superparamagnetic material causes in the applied magnetic field at each of the points 88, the number of point(s) 88 remaining embedded in the bearing 84 may be determined. It should be appreciated that the number of points 88 embedded in the tibial bearing decreases as a result of wear of the bearing 84. As such, by determining presence (or by inference, lack thereof) of the points 88, the degree of wear of the tibial bearing 84 may be correlated.

It should be appreciated that although the points 88 of the patterns 86 of the tibial bearing 84 are shown in FIG. 8 as being aligned vertically with one another, such an arrangement need not be the case. In particular, the points 88 may be arranged in any desired spatial relationship.

It should also be appreciated that the superparamagnetic patterns 86 may be visualized in a similar manner to as described above in regard to the barcodes of FIG. 1 (for example, by use of an MRI, an X-ray, and/or a custom measurement system utilizing one or more of the aforementioned highly sensitive magnetic sensors). The resultant film or digital rendering, (for example, digital X-ray) may then be analyzed manually (for example, visually) or by an automated device for determining the number of points 88 that remain embedded in the polymer bearing 84.

Although use of a superparamagnetic material to monitor wear of a knee bearing (that is, the tibial bearing 48) is herein described in regard to FIG. 8, it should be appreciated that such concepts may be used to monitor wear of any of the components of any type of prosthesis. For example, such concepts may be used in regard to the other components of a knee prosthesis, the components of a hip prosthesis (for example, an acetabular bearing), the components of a shoulder prosthesis (for example, a glenoid bearing), any of the components of a long bone prosthesis, spinal implants, or any other type of prosthesis.

Superparamagnetic materials may have numerous other uses in orthopaedic applications. For example, superparamagnetic materials which exhibit surface properties that allow the material to bind to bacteria are known. A coating having such a superparamagnetic material may be applied to an implantable prosthesis. A specific coating may be selected which allows for some degree of mobility of the superparamagnetic particles within the coating. If bacteria is present on the coated surface, the superparamagnetic particles with gather and bind to the bacteria. A detected increase in the concentration of superparamagnetic particles in a given area could be used as an indication of the presence of the bacteria. Such concentrations could be visualized using, for example, an MRI, an X-ray, and/or a custom measurement system utilizing one or more of the aforementioned highly sensitive magnetic sensors, or by monitoring distortion in an applied external magnetic field in any of the manners described above. In a similar concept, a superparamagnetic material and coating combination may be selected which allows the superparamagnetic material to bind to specific, predetermined molecular markers that are indicative of polyethylene wear.

Moreover, certain types of superparamagnetic materials, such as those having a high concentration of iron oxide, may be embedded in polymer implants such as polyethylene bearings. As a result of doing so, the polyethylene exhibits some degree of radiopaqueness. As such, the polyethylene bearing may be visualized on X-rays.

## Claims

1. An orthopaedic implant comprising a superparamagnetic material.

2. The orthopaedic implant of claim 1, further comprising an elongated nail having a hole defined therein.

3. The orthopaedic implant of claim 2, wherein a wall which defines the hole is coated with a composition comprising the superparamagnetic material.

4. The orthopaedic implant of claim 2, wherein the hole is at least partially filled with a composition comprising the superparamagnetic material.

5. The orthopaedic implant of claim 2, wherein the superparamagnetic material is positioned on an outer surface of the elongated nail at a location proximate to the hole.

6. The orthopaedic implant of claim 2, wherein the hole comprises a screw hole adapted to receive a bone screw.

7. The orthopaedic implant of claim 1, further comprising a polymer component, wherein the superparamagnetic material is secured to the polymer component.

8. The orthopaedic implant of claim 7, in which the polymer component comprises a polyethylene bearing, and the superparamagnetic material is embedded in the polyethylene bearing.

9. The orthopaedic implant of claim 1, further comprising a first component and a second component, in which at least one of the first component and the second component is movable relative to the other, and the superparamagnetic material is secured to both the first component and the second component.

10. The orthopaedic implant of claim 9, in which the first component comprises a tibial tray, and the second component comprises a tibial bearing.

11. The orthopaedic implant of claim 9, in which the first component comprises a tibial tray, and the second component comprises a femoral knee component.

12. The orthopaedic implant of claim 1, wherein the superparamagentic material is arranged in a pattern which defines a machine-readable indicia.

13. The orthopaedic implant of claim 12, wherein the machine-readable indicia comprises a barcode.

14. The orthopaedic implant of claim 1, in which the implant defines a thickness, and the superparamagnetic material is arranged in a pattern which changes as a result of changes in the thickness of the implant.
